# EUROPEAN PATENT SPECIFICATION

(11) **EP 4 312 911 B1**
(45) Date of publication and mention of the grant of the patent: **05.11.2025**
(21) Application number: 22721819.5
(22) Date of filing: 30.03.2022
(51) Int. Cl.: A61F 5/455

(54) **MENSTRUAL CUP**
MENSTRUATIONSBECHER
COUPELLE MENSTRUELLE

(30) Priority: 30.03.2021 GB 202104537
(43) Date of publication of application: 07.02.2024
(73) Proprietor: EMM Technology Ltd, Bristol BS1 6QA (GB)
(72) Inventor: VAN KEMPEN, Christopher, Bristol BS1 6QA (GB); BUTTON, Jennifer, Bristol BS1 6QA (GB)
(74) Representative: EIP
(86) International application number: PCT/GB2022/050797
(87) International publication number: WO 2022/208081

(56) References cited:
- EP-A1- 3 773 369
- DE-U1- 202017 106 300
- KR-B1- 101 915 316
- KR-B1- 102 083 431
- US-A1- 2014 222 122
- US-A1- 2020 046 572
- US-A1- 2021 069 009

## Description

### Field of the Invention

The present invention relates to a menstrual cup.

### Background of the Invention

In comparison to single-use products, such as tampons and sanitary pads, menstrual cups can provide a cost-effective and environmentally-friendly means for collecting menstrual fluid.

US2014222122 discloses antra-vaginal devices and methods are disclosed for a broad range of applications in the women's health field, including placement in the cervical region, and for transferring heat to a cervical region, a barrier contraception method, collection of menstrual fluid, and release of drugs and/or hormones intra-vaginally.

US2021/069009 discloses a menstrual cup that can be reused by replacing only a container containing menstrual blood. The menstrual cup comprises longitudinal corrugated portions having space portions formed therebetween by concave portions and convex portions such that removal can be facilitated by releasing the vacuum inside the vagina at the time of removal after use.

US2020046572 discloses a menstrual cup which includes a main body in which an opening is formed at one side, a storage configured to accommodate menstrual blood therein is formed, and an air inlet is formed at an upper end. An air flow path is formed between an outer wall and an inner wall of the main body and configured to connect the air inlet to the outside of the main body.

KR101915316 discloses an insertion type menstrual cup that includes: a main body having a cup shape, of which the upper part is open and of which the inner diameter gets narrower from top to bottom, to be inserted into the vagina to collect and store menstrual blood; and a grip extended downwards from the bottom of the body to induce the body in the vagina to easily collect the blood.

EP3773369 discloses an intravaginal device includes arms and struts. Each strut has a first end coupled to a shaft arranged to slide in a tube and a second end coupled to one of the arms. A string is coupled to the shaft. The arms have a stowed orientation in which the struts are folded inwards and a deployed orientation in which the struts point radially outwards and tautly hold the arms in an expanded position.

### Summary of the Invention

The present invention provides a menstrual cup comprising a main body to hold menstrual fluid, wherein the main body comprises a plurality of segments, adjacent segments are connected along a fold line, the cup is moveable between an expanded state and a collapsed state, the main body folds along the fold lines when the cup moves between the expanded and collapsed states, and the segments project outwardly beyond the fold lines when the cup is in the expanded state, herein the cup comprises a rim provided at an open end of the main body, and the rim has an outer edge having an elliptical profile in a radial direction when the cup is in the expanded state, and the outer edge is undulated in an axial direction when the cup is in the expanded state.

The segments may be arranged about a central longitudinal axis, and the segments may project radially outward beyond the fold lines when the cup is in the expanded state. That is to say that, in a plane normal to the longitudinal axis, the segments project radially outwardly beyond the fold lines.

Each fold line may have a maximum depth of at least 5 mm when the cup is in the expanded state. That is to say that, in a plane normal to a central longitudinal axis of the cup, a difference (measured at the exterior surface of the cup) between the radial distance of a fold line and the maximum radial distance of the segments connected by the fold line is at least 5 mm.

Each of the fold lines may have a depth that varies along the length of the main body. More particularly, each of the fold lines may have a depth that decreases in a direction towards a base of the main body. Consequently, each of the fold lines is deeper at a top of the main body, and shallower at a base of the main body.

The cup may comprise a central longitudinal axis and, when the cup is in the expanded state, each of the fold lines may have a maximum radial distance of R1, each segment may have a maximum radial distance of R2, and R2 may be greater than R1. Moreover, R2/R1 may be at least 1.2. Additionally or alternatively, R2-R1 may be at least 5 mm.

When the cup is in the expanded state, the main body may have a fold angle of no greater than 130 degrees at each of the fold lines. Moreover, the fold angle may be no less than 60 degrees. The fold angle may be around 90 degrees.

An exterior surface of the main body may undulate about a central longitudinal axis when the cup is in the expanded state. Consequently, the exterior surface of the main body may comprise peaks and troughs. Each of the fold lines may be located at a trough in the exterior surface.

The main body may comprise a base and a side wall upstanding from the base, the side wall may comprise the plurality of segments, and the exterior surface of the side wall may undulate about the longitudinal axis. Moreover, the undulation in the side wall may extend along a major portion of the side wall.

The main body may comprise a base and a side wall, the side wall may comprise the plurality of segments, and the segments may pivot relative to the base when the cup moves between the expanded and the collapsed states.

The main body may comprise between five and eight segments.

The main body may be resiliently biased to the expanded state.

The cup comprises a rim provided at an open end of the main body, and the rim has an outer edge having an elliptical profile in a radial direction when the cup is in the expanded state. More particularly, the outer edge of the rim may have an elliptical profile in a radial direction relative to a central longitudinal axis of the cup.

The rim may fold as the cup moves between the expanded and collapsed states. Moreover, the rim may fold along fold lines that are aligned radially with the fold lines in the main body.

The rim is undulated when the cup is in the expanded state. An outer edge of the rim undulates in an axial direction and has an elliptical profile in a radial direction.

The rim may be funnelled towards the main body when the cup is in the expanded state. More particularly, an outer edge of the rim may be located axially above the main body and radially beyond the main body.

The present disclosure also provides a menstrual cup comprising a main body to hold menstrual fluid, wherein the main body comprises a plurality of fold lines, the cup is moveable between an expanded state and a collapsed state, the cup is resiliently biased to the expanded state, the main body folds along the fold lines when the cup moves between the expanded and collapsed states, and each of the fold lines has a depth that varies along a length of the main body.

Each fold line may have a maximum depth of at least 5 mm when the cup is in the expanded state.

Each fold line has a depth that decreases towards a base of the main body. Consequently, each of the fold lines is deeper at a top of the main body, and shallower at a base of the main body.

The main body may comprise between five and eight fold lines.

When the cup is in the expanded state, the main body may have a fold angle of no greater than130 degrees at each of the fold lines.

The present disclosure further provides a menstrual cup comprising a main body to hold menstrual fluid, wherein the main body comprises between five and eight segments, adjacent segments are connected along a fold line, the cup is moveable between an expanded state and a collapsed state, and the main body folds along the fold lines when the cup moves between the expanded and collapsed states.

The present disclosure still further provides a menstrual cup comprising a main body to hold menstrual fluid, wherein the main body comprises a base and a side wall, the side wall comprises a plurality of segments, the cup is moveable between an expanded state and a collapsed state, and the segments pivot relative to the base when the cup moves between the expanded and the collapsed states.

The present disclosure additionally provides a menstrual cup comprising a main body to hold menstrual fluid, and a rim to engage a vaginal wall when the cup is inserted into a vagina, wherein the rim is provided at an open end of the main body, and the rim is undulated.

An outer edge of the rim may undulate in an axial direction and have an elliptical profile in a radial direction. That is to say that the outer edge may undulate in a direction parallel to a central longitudinal axis of the cup, and have an elliptical profile in a direction normal to the central longitudinal axis.

The rim may comprise a sequence of peaks and troughs arranged symmetrically about the rim.

The rim may comprise peaks and troughs, and the rim may have a length of at least 5 mm at each of the peaks or at each of the troughs.

The rim may be funnelled towards the main body. More particularly, an outer edge of the rim may be located axially above the main body and radially beyond the main body.

The cup may be moveable between an expanded state and a collapsed state, the cup may be resiliently biased to the expanded state, and the rim may be undulated when the cup is in the expanded state. Moreover, the rim may comprise peaks and troughs, and the rim may fold along one of the peaks and troughs when the cup moves between the expanded and collapsed states.

When the cup is in the expanded state, the rim may have an outer edge that undulates in an axial direction and has an elliptical profile in a radial direction.

The rim may have an inner edge and an outer edge, the outer edge may be one of above or below the inner edge when the cup is in the expanded state, and the outer edge may be the other of above or below the inner edge when the cup is in the collapsed state.

The main body may comprise a plurality of fold lines, the rim may comprise peaks and troughs, and one of the peaks and troughs may be aligned radially with the fold lines in the main body.

The rim may be annular.

The main body may be formed of an elastomeric material. Moreover, the elastomeric material may have a Shore hardness of between 30A and 50A.

The cup may comprise a frame embedded within the main body. The frame may comprise a plurality of ribs, each rib extending along a respective segment of the main body. The frame may additionally comprise a base from which the ribs extend, and the ribs may bend relative to the base when the cup moves between the expanded and collapsed states.

The frame may support or house an electronic assembly. For example, the frame may comprise a base and a plurality of ribs that extend from the base, and the electronic assembly may comprise a battery and a circuit assembly housed within the base. Additionally or alternatively, one or more of the ribs may support a sensor of the electronic assembly.

### Brief Description of the Drawings

Figure 1 is a perspective view of a first menstrual cup in an expanded state;
Figure 2 is a cross-sectional view through the first menstrual cup;
Figure 3 is a perspective view of the first menstrual cup in a collapsed state;
Figure 4 illustrates the behaviour of a fold in a rim of the first menstrual cup;
Figure 5 is a perspective view of a second menstrual cup;
Figure 6 is a perspective view of a third menstrual cup in an expanded state;
Figure 7 is a perspective view of the third menstrual cup in a collapsed state;
Figure 8 illustrates the behaviour of a fold in a rim of (a) the first menstrual cup, and (b) the third menstrual cup;
Figure 9 is a perspective view of a fourth menstrual cup;
Figure 10 is a perspective view of a fifth menstrual cup;
Figure 11 is a perspective view of a sixth menstrual cup;
Figure 12 is a perspective view of a seventh menstrual cup;
Figure 13 is a cross-sectional view through the seventh menstrual cup;
Figure 14 is a plan view of an eighth menstrual cup in (a) an expanded state and (b) a collapsed state;
Figure 15 is a plan view of ninth menstrual cup in (a) an expanded state and (b) a collapsed state;
Figure 16 is a perspective view of (a) a first frame, (b) a second frame, and (c) a third frame, each of which may be embedded within a main body of a menstrual cup;
Figure 17 is a cross-sectional view through a rib of a frame; and
Figure 18 shows use of a menstrual cup with an applicator.

### Detailed Description of the Invention

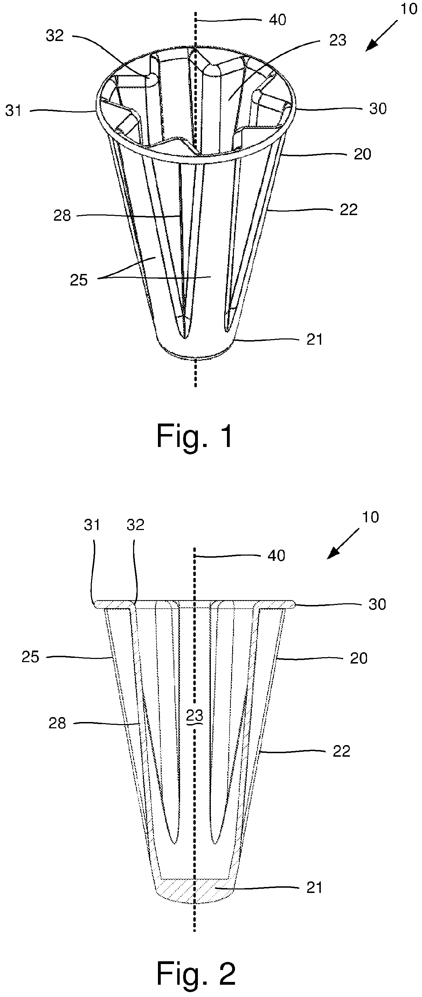

The menstrual cup 10 of Figures 1 to 3 is a single-piece component formed of an elastomeric material, such as silicone, rubber, TPE or the like. The menstrual cup comprises a main body 20 and a rim 30.

The main body 20 is generally conical in shape and comprises a base 21 and a side wall 22 upstanding from the base 21. The interior of main body 20 defines a chamber 23 for the collection of menstrual fluid.

The side wall 22 comprises a plurality of segments 25, which are arranged about a central longitudinal axis 40. Each of the segments 25 is identical in shape and is connected to adjacent segments along a fold line 28. In the example of Figure 1, each of the segments 25 has an exterior profile that resembles a wedge or doorstop, with the thin edge of the wedge adjoined to the base 21 of the main body 20. Each of the segments 25 projects outwardly beyond the fold lines 28. That is to say that, in a plane normal to the longitudinal axis 40, the segments 25 project beyond the fold lines 28 in a radial direction.

Each of the fold lines 28 has a depth that varies along the length of the side wall 22. More particularly, the depth of each of the fold lines 28 decreases in a direction towards the base 21 of the main body 20. Consequently, each of the fold lines 28 is deeper at a top and shallower at a bottom of the side wall 22.

Since the segments 28 project outwardly beyond the fold lines 28, the exterior surface of the side wall 22 is undulated. More particularly, the exterior surface undulates about the longitudinal axis 40. That is to say that, in a plane normal to the longitudinal axis 40, the exterior surface of the side wall 22 comprises peaks and troughs. The peaks correspond to the outermost surfaces the segments 25, and the troughs correspond to the fold lines 28. The undulation in the exterior surface of the side wall 22 continues along the majority of the side wall 22, i.e. from the base 21 to the rim 30.

As described below, the side wall 22 may be slightly thicker or thinner in areas, e.g. to increase the stiffness of the segments 25 or to better encourage folding along the fold lines 28. Nevertheless, the side wall 22 is generally of uniform thickness. Consequently, the interior surface of the side wall 22 is likewise undulated.

The rim 30 is provided at an open end of the main body 20. More particularly, the rim 30 is provided at a top of the side wall 22. The side wall 22 may therefore be said to extend between rim 30 and the base 21. When the menstrual cup 10 is inserted into the vagina of a user, the rim 30 engages with and forms a seal against a wall of the vagina. In the example of Figure 1, the rim 30 is flat and has an outer edge 31 that is circular in shape, and an inner edge 32 that corresponds to the undulating profile of the side wall 22. The outer diameter of the rim 30 corresponds to the outer diameter of the top of the side wall 22. Consequently, the rim 30 does not project beyond the main body 20. As described below, this may result in increased comfort when the cup 10 is inserted into the vagina.

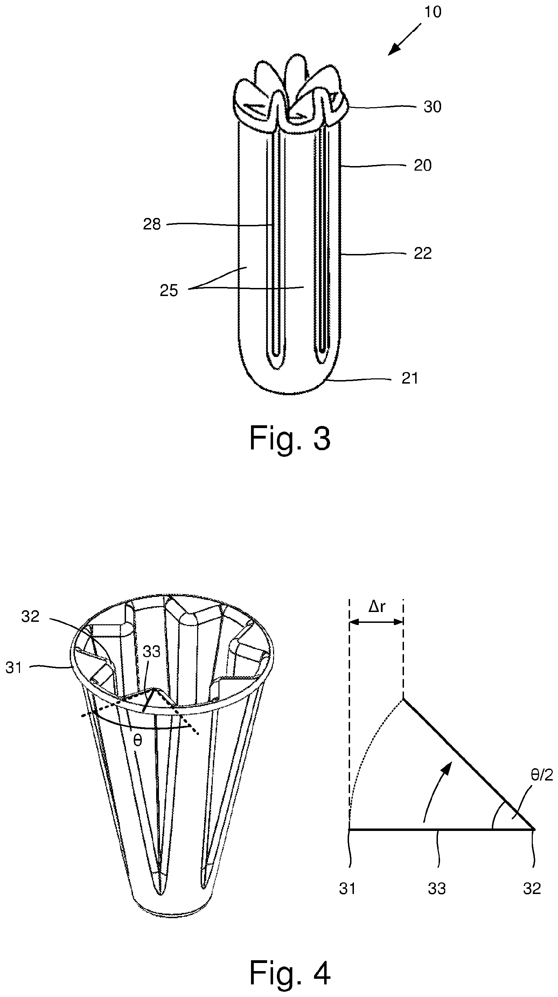

The menstrual cup 10 is moveable between an expanded state and a collapsed state. The expanded state is shown in Figure 1, and the collapsed state is shown in Figure 3.

As the menstrual cup 10 moves from the expanded state to the collapsed state, each of the segments 25 of the side wall 22 pivots inwardly from the base 21. Each of the segments 25 may therefore be regarded as being pivotally connected to the base 21. Additionally, the side wall 22 folds along each of the fold lines 28. The rim 30 also folds along lines that align with the fold lines 28 in the side wall 22. In particular, each fold line in the rim 30 lies in the same longitudinal plane as a fold line 28 in the side wall 22. In order to better encourage the rim 30 to fold, grooves or thinner-wall sections may be provided on the underside of the rim 30.

The menstrual cup 10 is formed of an elastomeric material and is resiliently biased to the expanded state. Accordingly, in the absence of any external forces, the menstrual cup 10 returns to the expanded state.

Prior to insertion, a user may grip (e.g. with thumb, index and middle fingers) around the side wall 22 of the cup 10 and squeeze. This then causes the cup 10 to move from the expanded state to the collapsed state. With the cup 10 in the collapsed state, the cup 10 may be inserted into the vagina. After insertion, the user's grip on the side wall 22 is released. The menstrual cup 10, being resiliently biased, then expands and attempts to return to the expanded state. Depending on the size, shape and strength of the vagina, the menstrual cup 10 may expand fully or only partially to the expanded state. The rim 30 then engages with a wall of the vagina to provide a seal between the cup 10 and the vagina.

When the menstrual cup 10 is in the expanded state, the rim 30 is flat and the outer edge 31 of the rim 30 has a circular profile. By contrast, when the menstrual cup 10 is in a partially expanded state (i.e. between the expanded and collapsed states), the rim 30 is undulated. Additionally, the outer edge 31 of the rim has a radial profile that conforms to the size and shape of the vagina. For example, the natural shape of the vagina may be elliptical or oval. Following insertion of the cup 10, the side wall 22 and the rim 30 unfold and expand. The rim 30 then contacts the wall of the vagina which opposes any further expansion in the rim 30. Since the side wall 22 is formed of a plurality segments 25 that are capable of pivoting independently of one another, the outer edge 31 of the rim 30 is not constrained to a circular profile. The outer edge 31 of the rim 30 therefore adopts a radial profile that conforms to the size and shape of the vagina. Accordingly, in this particular example, the outer edge 31 of the rim 30 adopts a profile that is elliptical or oval in the radial direction. That is to say that, when the menstrual cup is viewed along the central longitudinal axis, the rim appears elliptical or oval shaped.

The above description of the shape of the vagina is perhaps an oversimplification and the shape of the vagina is unlikely to be quite so regular or symmetric. Moreover, the size and shape of the vagina are not static but are instead dynamic. For example, the size and shape of the vagina may vary with contraction and relaxation of the pelvic floor muscles, e.g. due to physical activity or bowel movements. Irrespective of the size and shape which the vagina, the cup 10, and in particular the rim 30, are able to adopt a size and shape that conform. As a result, a comfortable and effective seal may be achieved between the cup 10 and the vagina.

With a conventional menstrual cup, the cup is typically folded prior to insertion. Various techniques exist for folding the cup, such as push-down, C-fold, 7-fold, S-fold etc. Some of the folding techniques (e.g. push-down, 7-fold or diamond-fold) result in a cup having a relatively small insertion point. However, the cup is relatively wide further down. As a result, whilst initial insertion of the cup into the vagina may be relatively comfortable, inserting the cup fully into the vagina can be uncomfortable. Other folding techniques (e.g. E-fold or S-fold) result in a relatively narrow cup along the full length of the cup. However, it can be difficult to maintain the cup in the folded configuration whilst inserting the cup into the vagina. Moreover, should the cup inadvertently unfold during insertion, the cup can slap against sensitive tissue.

When the menstrual cup 10 described above is in the collapsed state, as shown in Figure 3, the cup 10 presents a relatively small insertion point. Additionally, the cup 10 is relatively narrow along the full length of the cup 10. Furthermore, the cup 10 may be maintained in the collapsed state relatively easily using one hand. As a result, the menstrual cup 10 may be inserted more easily and/or with less discomfort than that of a conventional cup.

A further difficulty with conventional menstrual cups is that, once inserted, it can be difficult for the folded cup to return its original shape within the vagina. Moreover, the cup may abruptly return to its original shape with a pop, which can cause discomfort. Additionally, a user is typically required to check (e.g. by running a finger around the rim of the cup) that the cup is properly seated within the vagina and that an effective seal has been formed. With the menstrual cup 10 described above, on the other hand, unfolding or expansion of the cup 10 is both simpler and potentially more comfortable.

Conventional menstrual cups also suffer from the problem that the cup must be appropriately sized for each user. If the diameter of the cup is too small, the cup is unlikely to form an effective seal with the vaginal wall and may leak. If the diameter of the cup is too large, the rim of the cup may buckle and fail to form an effective seal. Additionally or alternatively, the cup may apply excessive pressure to the vaginal wall resulting in discomfort. Conventional menstrual cups are therefore often provided in a range of different sizes. A user is then required to guess the appropriate size based on factors such as age, the position of the cervix, whether the user has previously given birth, the level of activity of the user, and volume of flow.

With the menstrual cup 10 described above, the rim 30 of the cup 10 adapts to the size and shape of the vagina of the user. As a result, a single cup may be used to provide effective sealing over a range of different sizes and shapes without the discomfort or reliability issues that might otherwise arise from an incorrectly sized cup.

As already noted, the size and/or shape of the vagina are dynamic and may vary during use of a menstrual cup, e.g. due to contraction and expansion of the pelvic floor muscles. With a conventional menstrual cup, changes in the size and/or shape of the vagina may result in leaking. With the menstrual cup 10 described above, however, the cup 10 moves in response to changes in the size and/or shape of the vagina such that an effective seal continues to be provided. The menstrual cup 10 therefore provides a dynamic seal, i.e. one that moves and adapts in response to changes in the size and/or shape of the vagina.

The menstrual cup 10 may be removed in a similar way to that of a conventional cup. In particular, a user may reach up and grip the main body 20. The side wall 22 may then be squeezed or pinched in order to break the seal between the rim 30 and the vaginal wall. Although not shown in the Figures, the menstrual cup 10 may comprise a stem, similar to that found on conventional menstrual cup, which extends downwardly from the main body 20. The stem may then be used to gently pull down the menstrual cup 10 to a point at which the user is able to reach up and grip the main body 20.

Removal of the cup 10 may be easier than that of a conventional cup. With a conventional menstrual cup, a user is typically required to reach deep inside the vagina to pinch the top of the cup in order to break the seal formed by the rim. However, reaching deep inside the vagina can be uncomfortable. Additionally, the user is more likely to interact with menstrual fluid on the vaginal wall. By contrast, with the menstrual cup 10 of Figure 1, the seal may be broken by pinching a lower part of the cup 10. In particular, squeezing or pinching the lower part of the side wall 22 may cause the upper part of the side wall 22 to fold inwardly, thereby causing the rim 30 to be pulled away from the vaginal wall to break the seal. The user is therefore able to break the seal by gripping the cup 10 at a position much closer to the vaginal opening. As a result, the cup 10 may be removed more ergonomically, and the user is less likely to interact with menstrual fluid. In order to better encourage this behaviour, each of the segments 25 may be stiffened such that pressing a lower part of the segment 25 causes the upper part of the segment 25 to move. Stiffening of the segments 25 may be achieved through the shape or geometry of the segments 25, thickening of the walls of the segments 25, and/or by including an internal frame within the main body 20.

Owing to the seal that is created between a menstrual cup and the vaginal wall, the cup entraps an enclosed volume defined by the vaginal wall, cervix and uterus. If the menstrual cup were pulled without first breaking the seal, the resulting suction could cause organ prolapse or dislodge intrauterine devices. Owing to the recognised difficulty in removing conventional menstrual cups, the cups typically include one or more small air holes located just below the rim. Whilst the holes reduce the suction should the cup be pulled without first breaking the seal, the holes provide a leak path for menstrual fluid and are difficult to clean hygienically. With the menstrual cup of Figure 1, the need for such holes is averted owing to the ease by which the cup 10 can be collapsed and the seal broken.

As noted above, when the menstrual cup 10 is in a partially expanded state, the rim 30 is not flat but is instead undulated. More particularly, the outer edge 31 of the rim 30 undulates in the axial direction, i.e. in a direction parallel to the longitudinal axis 40 of the cup 10. In spite of the axial undulation in the outer edge 31 of the rim 30, there is relatively little radial undulation. This then ensures that an effective seal is formed between the rim 30 and the vagina at different sizes and shapes. As explained below, key to this behaviour is the notion that, as the size and/or shape of the rim 30 changes, the rim 30 folds in such a way that the outer edge 31 of the rim 30, at each fold, moves axially.

For the purposes of the present discussion, let us consider a simplified example in which the desired shape of the rim 30 is circular. As the cup 10 moves between the expanded and collapsed states, the rim 30 ideally folds in such a way that the outer edge 31 of the rim 30 maintains a circular profile in the radial direction. As noted, key to this behaviour is the notion that, as the outer diameter of the rim 30 changes, the rim 30 folds in such a way that the outer edge 31 of the rim 30, at each fold, moves axially.

Figure 4 illustrates the behaviour of the rim 30 at each fold line 33 in the rim 30. Each fold line 33 extends between a point on the outer edge 31 of the rim 30 and a point on the inner edge 32. As the rim 30 folds, the fold line 33 pivots about the point on the inner edge 32, thereby causing the point on the outer edge 31 to move. In the particular example shown in Figure 4, the point on the outer edge 31 moves in an upward direction. However, as described further below, the point on the outer edge 31 could conceivably move in a downward direction. Since the fold line 33 pivots, the point on the outer edge 31 of the rim 30 moves along an arcuate path. Consequently, as the rim 30 folds, movement of the outer edge 31 is not purely axial, as desired, but instead has a radial component, Δr. As a result, the radial profile of the outer edge 31 of the rim 30 is not perfectly circular but instead comprises a radial undulation, Δr. The size of this radial component is, however, relatively small and thus the outer edge 31 of the rim 30 nevertheless maintains a substantially circular profile at different outer diameters.

As can be seen in Figure 4, as the rim 30 folds, the outer edge 31 moves along an arcuate path having a radius of curvature defined by the length of the fold line 33. The radial undulation, Δr, in the outer edge 31 of the rim 30 is therefore defined by the length of the fold line 33. In particular, as the length of the fold line 33 increases, the degree of radial undulation decreases. It is therefore beneficial to have fold lines 33 in the rim 30 that are relatively long. The length of each fold line 33 in the rim 30 is defined in part by the depth of the fold lines 28 in the side wall 22. The fold lines 28 in the side wall 22 are therefore relatively deep, even when the cup 10 is in the expanded state, so as to increase the length of the fold lines 33 in the rim 30. However, as the depth of the fold lines 28 in the side wall 22 increases, the capacity of the cup 10 decreases. There is therefore a balance to be struck between the desire to reduce radial undulation in the outer edge 31 of the rim 30 (so as to achieve good sealing for different sizes and shapes of vagina), and the desire to maximise the capacity of the cup. By employing a rim having a length of at least 5 mm at each fold line in the rim, radial undulation in the outer edge of the rim may be kept relatively small over a good range of different sizes and shapes, e.g. outer diameters of between 35 mm and 48 mm. Furthermore, a relatively good balance between the competing factors of radial undulation and capacity may be achieved with a rim having a length of between 5 mm and 12 mm at each fold line. As already noted, the length of each fold line 33 in the rim 30 is defined in part by the depth of the fold lines 28 at the top of the side wall 22. Accordingly, the depth of the fold lines 28 at the top of the side wall 22 may be at least 5 mm and more particularly between 5 mm and 12 mm.

When the cup 10 is in the expanded state, each of the fold lines 28 may be said to have a maximum radial distance of R1 relative to the longitudinal axis 40. Additionally, each segment 25 may be said to have a maximum radial distance of R2. R2 is then greater than R1 and thus the segments 25 project radially outward beyond the fold lines 28 when the cup 10 is in the expanded state. The ratio of R2/R1 may be regarded as a relative measure of the depth of the folds lines 28 in the side wall 22. In particular, a higher R2/R1 ratio implies a deeper fold line 28. For the reasons set out in the previous paragraph, the ratio of R2/R1 may be at least 1.2. Moreover, the difference between R2 and R1 (i.e. R2-R1) may be at least 5 mm.

It can also be seen in Figure 4 that the degree of radial undulation in the outer edge 31 of the rim 30 is defined by the fold angle θ in the side wall 22 (i.e. the angle between adjacent segments 28 when measured at a fold line 28), when the cup 10 is in the expanded state. In particular, as the fold angle θ increases, the degree of radial undulation, Δr, increases. Accordingly, when the cup 10 is in the expanded state, the side wall 22 may have a fold angle of no greater than 130 degrees at each of the fold lines 28. As the fold angle decreases, the available space into which the rim 30 folds is reduced and therefore higher strains may arise. The fold angle may therefore be no less than 60 degrees, when the cup 10 is in the expanded state. More particularly, a good balance between these competing factors may be found with a fold angle of around 90 degrees.

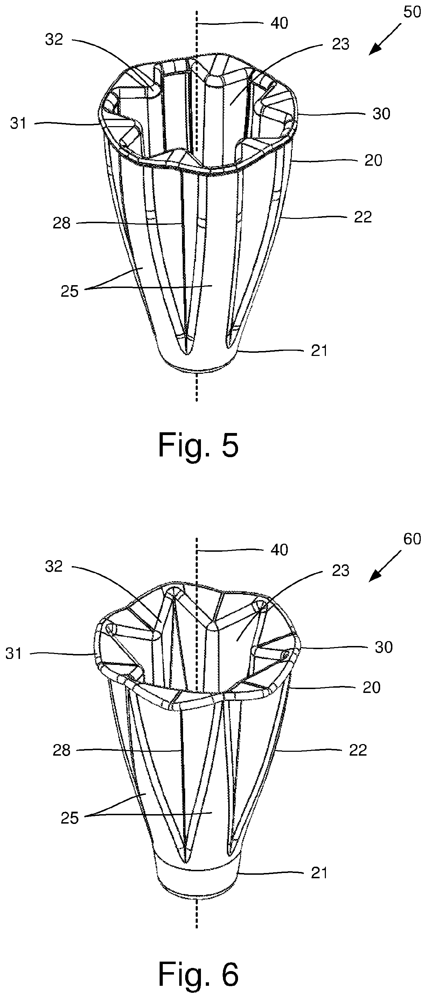

Figure 5 illustrates an alternative menstrual cup 50. The menstrual cup 50 is identical in many respects to that shown in Figure 1. However, the cup 50 differs in two respects: the shape of the main body 20, and the shape of the rim 30.

With the cup 10 shown in Figure 1, the main body 20 is generally conical in shape, with the outer diameter of the side wall 22 tapering or decreasing linearly from the top to the bottom of the side wall 22. By contrast, the main body 20 of the cup 50 of Figure 5 is more bell shaped. The upper portion of the side wall 22 is straighter (i.e. the outer diameter is relatively constant), and the lower portion of the side is tapered (i.e. the outer diameter decreases). Providing a straighter upper portion to the main body 20 has at least two advantages. First, the capacity of the cup 50 is increased. Second, the alignment and stability of the cup 50 within the vagina may be improved. For example, the straighter upper portion may help prevent rocking or tilting of the cup 50 within the vagina after insertion. Additionally, the cup 50 may be more effectively gripped by the vagina wall, thereby providing a more secure placement. However, by having a straighter upper portion, the cup 50 is less compact when in the collapsed state. Additionally, since each fold line 28 in the side wall 22 is now curved, greater strains may arise during folding and thus a larger compressive force may be required to move the cup 50 from the expanded state to the collapsed state.

With the menstrual cup 10 of Figure 1, the rim 30 is flat when the cup 10 is in the expanded state. By contrast, the rim 30 of the cup 50 of Figure 5 is undulated when the cup 50 is in the expanded state. More particularly, the outer edge 31 of the rim 30 undulates in the axial direction, i.e. in a direction parallel to the longitudinal axis 40. The outer edge 31 of the rim nevertheless has a circular profile in the radial direction, i.e. in a direction normal to the longitudinal axis 40. The rim 30 may therefore be thought of as a pre-folded or partially collapsed form of the rim of Figure 1.

The undulation in the rim 30 is such that the rim 30 has peaks that are aligned radially with the fold lines 28 in the side wall 22. That is to say that each peak in the rim 30 lies in the same longitudinal plane as a fold line 28 in the side wall 22. Consequently, as the menstrual cup 50 moves from the expanded state to the collapsed state, the rim 30 folds along each of the peaks. Moreover, the rim 30 folds upwardly at each of the peaks. As a result, the folds in the rim 30 are clear of the side wall 22 and thus a more compact arrangement may be achieved when the cup 50 is in the collapsed state. By contrast, if the rim 30 were to fold downwardly, the folds in the rim 30 would be sandwiched between adjacent segments 25 of the side wall 22, resulting in a less compact arrangement.

Figure 5 shows the menstrual cup 50 in the expanded state. The menstrual cup 50 resembles that shown in Figure 3 when in the collapsed state. By providing an undulated or pre-folded rim, movement of the menstrual cup 50 from the expanded state to the collapsed state may be made easier. Moreover, the rim 30 folds in a more controlled manner. In this particular example, the rim 30 folds upwardly at each of the folds or peaks in the rim. By contrast, with the cup 10 of Figure 1, the rim 30 could conceivably fold upwardly or downwardly at each of the folds. With the cup 10 of Figure 1, grooves or thinner-wall sections may be provided in the rim 30 in order to better encourage the rim 30 to fold in a particular direction. However, the rim 30 may then be more susceptible to tearing. With the menstrual cup 50 of Figure 5, a thicker rim and/or a rim of uniform thickness may be employed, thus reducing the risk of tearing. Furthermore, by providing a rim 30 that is undulated or pre-folded, the rim 30 is required to bend through a smaller angle at each fold. As a result, the strains experienced by the rim 30 at each fold are reduced and thus the likelihood of tearing may be further reduced.

In addition to the undulation, the rim 30 of the cup 50 of Figure 5 differs from that of Figure 1 in that it extends radially beyond the main body 20, which is to say that the outer diameter of the rim 30 is greater than that at the top of the side wall 22. By having a rim 30 that projects beyond the main body 20, the interference between the cup 50 and the vagina wall is better concentrated at the rim 30, thereby potentially improving the seal. However, the pressure applied by the cup 50 to the vaginal wall is then concentrated over a smaller contact area. With the cup 10 of Figure 1, on the other hand, the pressure applied by the cup 10 to the vaginal wall is distributed over a larger area, namely the rim 30 and the upper part of the side wall 22, which may result in improved comfort.

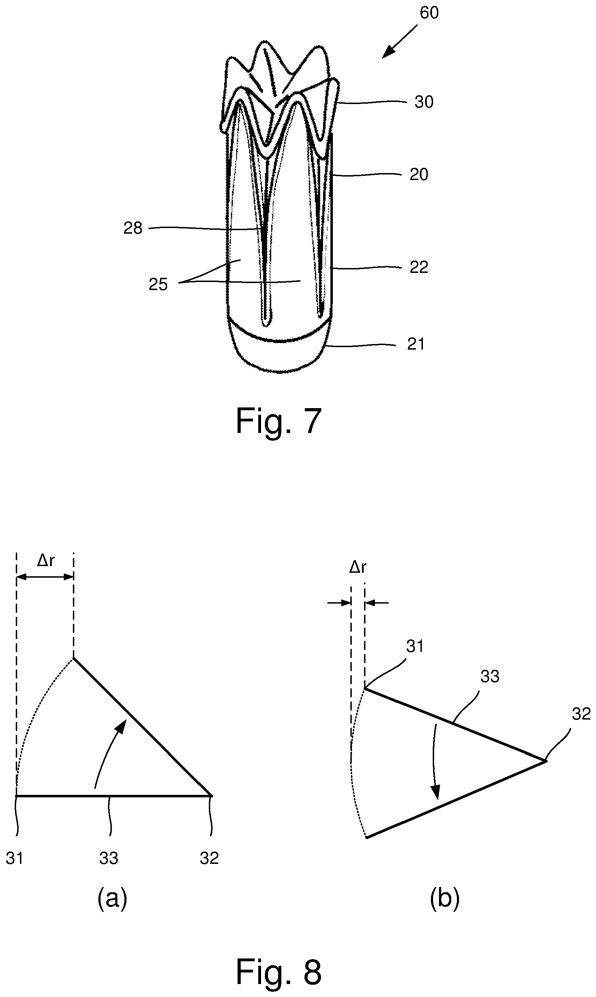

Figures 6 and 7 illustrate a further menstrual cup 60 in the expanded state (Figure 6) and the collapsed state (Figure 7). Again, the menstrual cup 60 is identical in many respects to those of Figures 1 and 5. However, the base 21 of the main body 20 is longer, which may aid in the removal of the menstrual cup 60 by providing a larger portion for the user to grasp. The base 21 may be ribbed or comprise other surface features to assist with gripping.

A further difference between the cup 60 of Figure 6 and those of Figures 1 and 5 resides in the shape of the segments 25 of the side wall 22. With the cups 10,50 of Figures 1 and 5, the outermost surface of each segment 25 is rectangular in shape. With the cup 60 of Figure 6, on the other hand, the outermost surface of each segment 25 is triangular in shape and tapers to a point at the top of the side wall 22. This then has the advantage that the cup 60 is capable of achieving a more compact size when in the collapsed position.

The rim 30 of the cup 60 again undulates and comprises peak and troughs. However, in contrast to the cup 50 of Figure 5, it is the troughs rather than the peaks that are aligned radially with the fold lines 28 in the side wall 22. Consequently, as the menstrual cup 60 moves to the collapsed state, the rim 30 folds downwardly rather than upwardly. As noted above, this may result in a less compact arrangement when the menstrual cup is in the collapsed configuration. However, in this particular example, this is offset by the gains made by having triangular-shaped segments 25. Moreover, in this particular example, having a rim 30 that folds downwards has the benefit of reducing the radial undulation, Δr, in the outer edge 31 of the rim 30. This is because, for this particular cup 60, the outer edge 31 of the rim 30, at each fold line 33, is above the inner edge 32 when the cup 60 is in the expanded state, and the outer edge 31 is below the inner edge 32 when the cup 60 is in the collapsed state. This behaviour is illustrated in Figure 8, in which the radial undulation, Δr, in the outer edge 31 of the rim is shown for (a) the cup 10 of Figure 1, and (b) the cup 60 of Figure 6. With the cup 10 of Figure 1, shown in Figure 8(a), the outer edge 31 of the rim is at the same height as the inner edge 32 when the cup is in the expanded state, and the outer edge 31 of the rim is above the inner edge 32 of the rim when the cup is in the collapsed state. With the cup 60 of Figure 6, shown in Figure 8(b), the outer edge 31 of the rim is above the inner edge 32 when the cup is in the expanded state, and the outer edge 31 of the rim is below the inner edge 32 when the cup 60 is in the collapsed state. It can be seen that the radial undulation, Δr, observed in Figure 8(b) is significantly smaller than that in Figure 8(a). The same behaviour would, of course, be observed for a cup in which the outer edge of the rim is below the inner edge when the cup is in the expanded state, and the outer edge of the rim is above the inner edge when the cup is in the collapsed state. Accordingly, in a more general sense, a reduction in the radial undulation in the outer edge of the rim may be achieved by having a rim in which, at each fold, the outer edge is one of above or below the inner edge when the cup is in the expanded state, and the outer edge is the other of above or below the inner edge when the cup is in the collapsed state.

Returning to Figure 6, the rim 30 of the cup 60 is funnelled towards the main body 20 when the cup 60 is in the expanded state. More particularly, the rim 30 projects radially outward and axially upward from the side wall 22 of the main body 20. Consequently, the outer edge 31 of the rim 30 is located axially above and radially beyond the main body 20. As a result, the rim 30 is better able to collect and guide menstrual fluid into the interior chamber 23 of the cup 60.

As noted above, the outermost surface of each segment 25 of the cup 60 of Figure 6 is triangular in shape and tapers to a point at the top of the side wall 22. This then has the advantage that the cup 60 is relatively compact when in the collapsed position. However, when the cup 60 is inserted into the vagina, the upper portion of the side wall 22 of the cup 60 is likely to engage with and apply pressure to the vaginal wall. Since the outermost surface of each segment 25 tapers to a point at the top, the pressure to the vaginal wall is potentially applied over a relatively small contact area, which may be felt or discerned by a user.

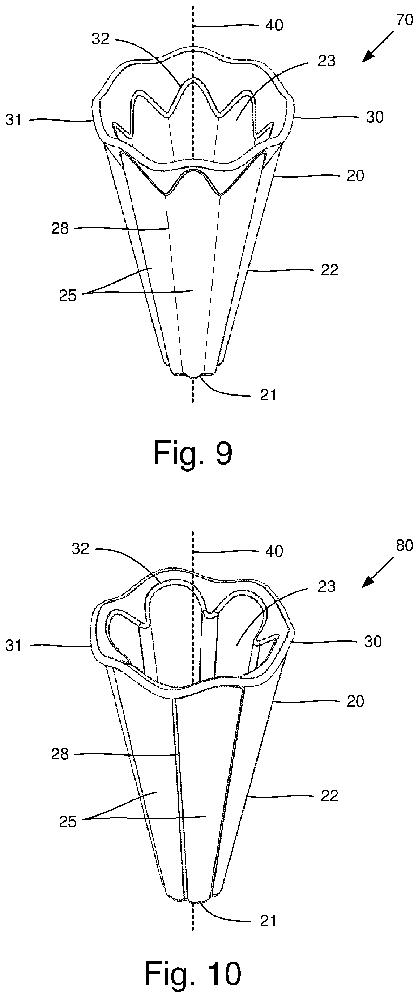

Figures 9 and 10 show two further cups 70,80 that address this potential shortcoming in the cup 60 of Figure 6. The exterior surface of the segments 25 of the side wall 22 are curved. As a result, the pressure applied to the vaginal wall by the side wall 22 is distributed over a larger area.

With the cup 70 shown in Figure 9, the side wall 22 undulates sinusoidally. The fold lines 28 between segments 25 are then defined by the troughs in the side wall 22, i.e. each fold line 28 corresponds to the nadir of a respective trough. An advantage of having a sinusoidal side wall 22 is that, as the cup 70 moves to the collapsed state, the compressive and tensile strains are no longer concentrated along distinct fold lines 28 but are instead distributed over a larger area. As a result, a smaller compressive force is required to move the cup 70 to the collapsed state. A potential difficulty with the cup 70, however, is that, the cup 70 is more likely to buckle or fold in an uncontrolled manner as it moves from the expanded state to the collapsed state. This is because, at least for the example shown in Figure 9, the fold lines 28 in the side wall 22 are less distinct, and the fold angle is relatively large. This may be mitigated by increasing the amplitude of the sinusoid in the side wall 22. As a result, the troughs or fold lines 28 in the side wall 22 are pushed further inward (i.e. are deeper), which in turn better encourages the side wall 22 to fold along the fold lines 28. However, by pushing the fold lines 28 further inward, the capacity of the cup 70 is reduced.

With the cup 80 shown in Figure 10, the segments 25 are again curved. However, adjacent segments 25 are connected along distinct fold lines 28 having a smaller fold angle. Consequently, as the cup 80 moves to the collapsed state, buckling or uncontrolled folding of the side wall 22 is less likely. However, the strains in the side wall 22 are now concentrated along the fold lines 28.

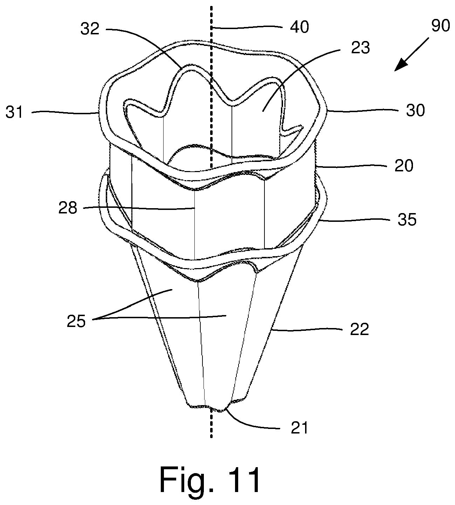

Figure 11 shows a further example of a menstrual cup 90. The cup 90 is identical to that of Figure 6 with two exceptions. First, the upper portion of the side wall 22 is straight, i.e. the outer diameter of the upper portion of the side wall 22 is constant. Second, the cup 90 comprises an additional rim 35 that projects outwardly from the side wall 22. More particularly, the additional rim 35 projects outwardly from the bottom of the upper portion of the side wall.

As noted above in connection with the cup 50 of Figure 5, providing a straight upper portion has at least two advantages. First, the capacity of the cup 90 is increased. Second, the alignment and stability of the cup 90 within the vagina may be improved. However, by having a straight upper portion, the cup 90 is potentially less compact when in the collapsed state. Additionally, since each fold line 28 is no longer linear, greater strains in the side wall 22 may arise. As a result, the side wall 22 may buckle or fold in an uncontrolled manner, and/or a larger compressive force may be required to move the cup 90 from the expanded state to the collapsed state.

The additional rim 35 engages with the vaginal wall to form a further seal between the cup 90 and the vagina. The additional rim 35 may therefore act to retain any menstrual fluid which has inadvertently leaked past the top rim 30. In spite of this potential advantage, the provision of the additional rim 35 is likely to increase the stiffness of the cup 90 in the region around the additional rim 35, which in turn may adversely affect how cup collapses. In particular, a larger force may be required to move the cup 90 to the collapsed state and/or the cup 90 may be more likely to buckle or fold in an uncontrolled manner.

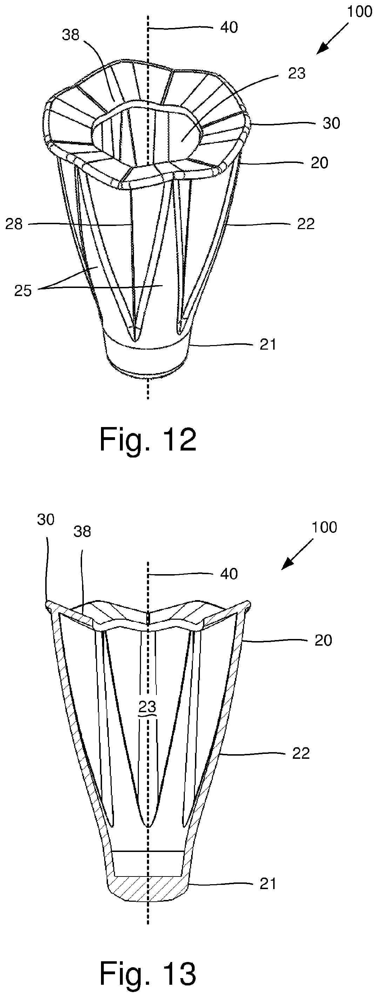

Figures 12 and 13 illustrate a menstrual cup 100 which is identical in all but one respect to the cup of Figure 6. The cup 100 differs from that of Figure 6 in that the cup comprises an anti-spill lip 38. The lip 38 is provided towards the top of the main body 20 and extends radially inward. In the example shown in Figures 12 and 13, the lip 38 extends radially inward from the side wall 22, such that the lip 38 appears to be an inward extension of the rim 30. The lip 38, like the rim 30, undulates. Moreover, the undulation in the lip 38 mirrors or corresponds to that of the rim 30. The peaks and troughs in the lip 38 are therefore aligned radially with the peaks and troughs in the rim 30 such that, as the cup 100 moves between the expanded and collapsed states, the lip 38 and the rim 30 fold along identical planes.

In extending radially inward, the lip 38 provides an anti-spill feature that helps minimise spillage of menstrual fluid during the removal of the cup 100. The lip 38 may, however, adversely affect the folding of the rim 30 and/or the side wall 22 as the cup 100 moves between the expanded and contracted states. In order to mitigate this, the lip 38 may comprise one or more radial splits or cuts. For example, a radial split or cut may be provided at each trough such that, rather than being continuous, the lip comprises a number of discrete sections. The lip would continue to provide a degree of anti-spill protection without necessarily adversely influencing the folding of the rim and/or side wall. In another example, the lip 38 may again comprise a plurality of discrete sections, with each section filling an undulation in the inner surface of the side wall 22 such that the top of the cup 100 appears to have a circular inner edge.

In addition to or as an alternative to the anti-spill lip 38, the top of the cup 100 may be slanted or angled relative to the longitudinal axis 40 such that side wall 22 is taller along one side. The cup 100 may then be inserted into the vagina such that the longer length of the side wall 22 is positioned towards the rear of the vaginal wall. This may then reduce potential spillage during removal of the cup 100.

Each of the menstrual cups described above and illustrated in the Figures comprises six segments 25 and six fold lines 28 in the side wall 22. The cups may, however, comprise an alternative number of segments 25 and fold lines 28.

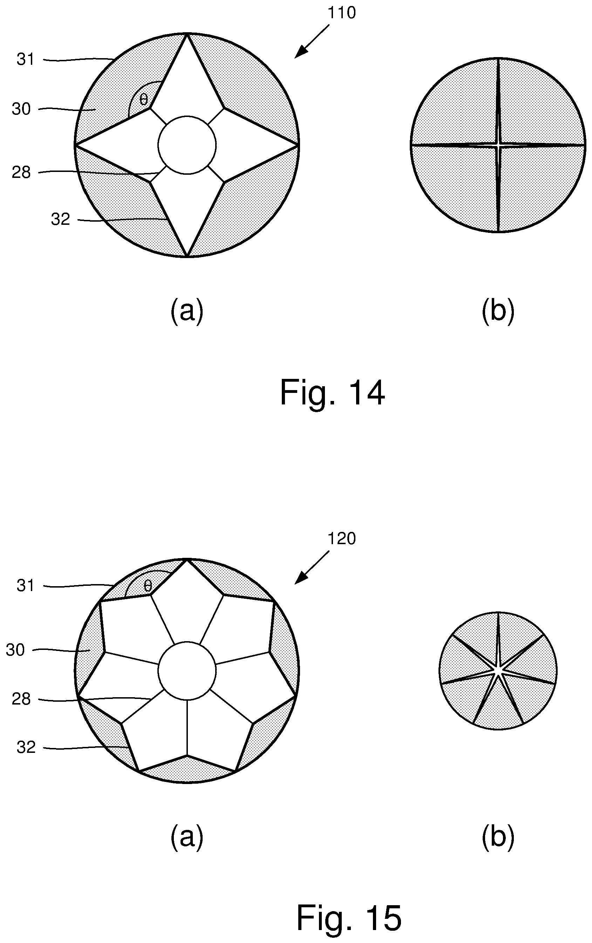

Figure 14 shows a plan view of a cup 110 having four segments. Figure 14(a) shows the cup 110 in the expanded state, and Figure 14(b) shows the cup 110 in the collapsed state. Figure 15 shows a plan view of a further cup 120 having seven segments. Again, Figure 15(a) shows the cup 120 in the expanded state, and Figure 15(b) shows the cup 120 in the collapsed state. The outer diameters of the two cups 110,120 are the same when in the expanded state. Additionally, the fold angles θ (i.e. the angle between adjacent segments when measured at a fold line) of the two cups 110,120 are the same in the expanded state and are approximately 140 degrees. It can be seen that, when the cups are in the expanded state, the capacity (i.e. the interior volume) of the cup 120 of Figure 15(a) is significantly larger than the capacity of the cup 110 of Figure 14(a). Furthermore, it can be seen that, when the cups are in the collapsed state, the size or outer diameter of the cup 120 of Figure 15(b) is significantly smaller. The cup 120 of Figure 15 therefore has the advantage of having a larger capacity when in the expanded state, and a smaller size when in the collapsed state. However, when the cups are in the collapsed state, the fold angle θ of the cup 110 of Figure 14(b) is around 90 degrees, a change of 50 degrees from the expanded state. By contrast, the fold angle θ of the cup 120 of Figure 5(b) is around 50 degrees, a change of 90 degrees from the expanded state. The cup 120 of Figure 15 therefore experiences greater strains when in the collapsed state. A greater compressive force is then required to move or squeeze the cup 120 of Figure 15 to the collapsed state. Additionally, the cup 120 may buckle or fold in an uncontrolled manner, and the greater strains may increase the likelihood of tearing.

Figures 14 and 15 are simplified representations of the cups 110, 120 and do not, for example, show the radial undulation in the outer edge of the rim 30 when the cups 110,120 are in the collapsed state. In addition to the capacity, the collapsed size and ease of use, the number of segments and the size of the fold angles also influence the amount of radial undulation in the outer edge of the rim 30, and thus the effectiveness of the cup 110,120 to form a good seal with the vagina.

The number of segments is therefore a balancing act of several factors, including the desire to maximise the capacity of the cup in the expanded state, the desire to minimise the size of the cup in the collapsed state, the desire to make it easy to move the cup from the expanded state to the collapsed state, and the desire to maintain an effective seal over different size and shapes. The number of segments may also depend on the size of the cup, the thickness of the side wall and the choice of elastomer (particularly the hardness or elasticity of the elastomer). A relatively good balance of the various factors may be achieved when using between five and eight segments. More particularly, a cup having six segments was found to provide a particularly good balance of capacity when expanded, compactness when collapsed, and ease of use, particularly for a cup formed of silicone, having a maximum outer diameter of between 35mm and 48 mm, and a side wall thickness of between 1.0 mm and 2.0 mm.

In each of the examples described above, the outer edge 31 of the rim 30 has a circular profile when the cup is in the expanded state. Additionally, the cup is rotationally symmetrical about the central longitudinal axis 40. This then has the advantage that the cup may be inserted into the vagina in any orientation. Nevertheless, for some women, improved comfort and/or sealing may be achieved with a cup having a more eccentric profile when in the expanded state. Accordingly, in a more general sense, the outer edge 31 of the rim 30 may be said to have an elliptical profile (circular or non-circular) when in the expanded state.

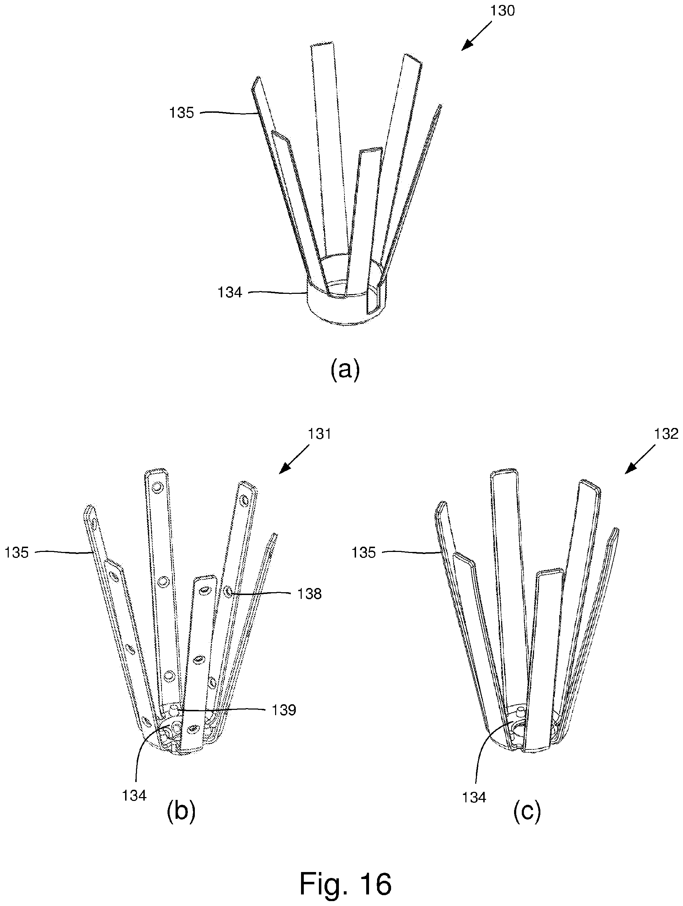

Each of the menstrual cups described above may comprise a frame embedded within the main body 20. Figure 16 illustrates three examples of a frame 130,131,132. Each of the frames 130 (hereafter only reference numeral 130 will be used) comprises a base 134 and a plurality of ribs 135 or fingers that extend upwardly from the base 134. When embedded within the main body 20, the base 134 of the frame 130 is located within the base 21 of the main body 20, and each of the ribs 135 extends along the length of a respective segment 25. The frames 130 illustrated in Figure 16 are intended for use with the cup 10 of Figure 1. The ribs 135 are therefore rectangular in shape, so as to correspond to the shape of the segments 25. The ribs 135 may, however, have an alternative shape and need not correspond to the shape of the segments 25. By way of example, should a frame be employed with the cups 60,100 of Figures 6 and 12, the ribs 135 may be triangular in shape.

When moving the menstrual cup from the expanded state to the collapsed state, the ribs 135 bend relative to the base 134 of the frame 130. In the examples illustrated in Figure 16, each of the ribs 135 is straight along its length. Accordingly, when the cup is moved to the collapsed state, the ribs 135 and the main body 20 present a relatively small cross-sectional area for insertion into and removal from the vagina. In other examples, the ribs 135 may have alternative shapes. For example, a frame having curved ribs may be used with the cup 50 of Figure 5, and a frame having angled ribs may be used with the cup 90 of Figure 11.

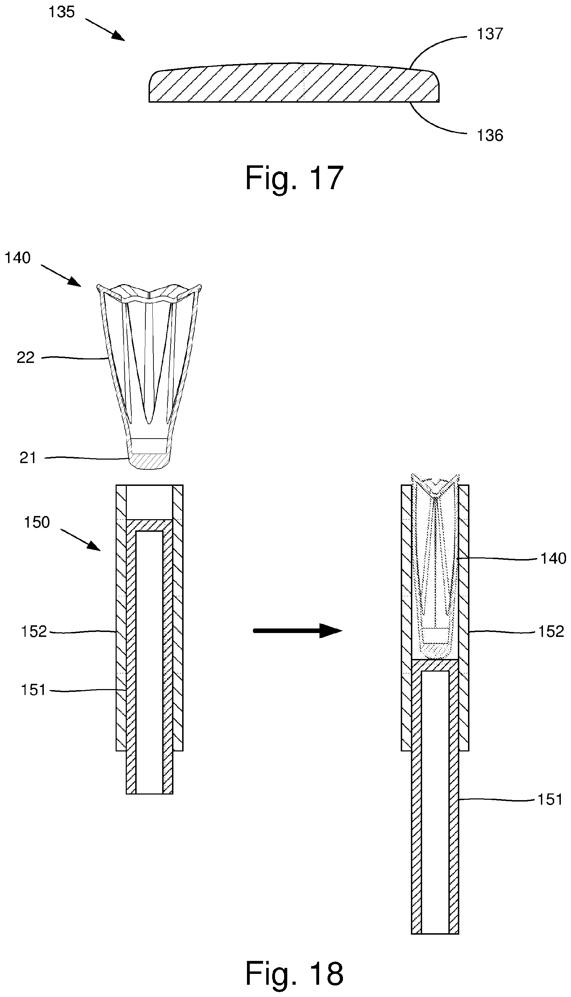

Figure 17 illustrates a cross-sectional view through an example of a rib 135. As noted in the preceding paragraph, the ribs 135 bend relative to the base 134 of the frame 130 when moving between the expanded and collapsed states. The inner surface 136 of each rib is therefore flat, at least at the point where the rib 135 joins the base 134. As a result, bending of the ribs 135 relative to the base 134 is better controlled and is made easier, and excessive stress concentrations may be avoided. The outer surface 137 of each rib is curved and the outwardly-facing corners and edges of the rib 135 are rounded so as to improve user comfort. Nevertheless, ribs 135 having alternative shapes and/or features may be employed. For example, in order to better encourage each rib 135 to bend at the base 134 of the frame 130 rather than, say, at a point along the length of the rib 135, the bottom of each rib 135 may comprise a localised reduction in wall thickness or some other hinge feature.

The frame 130 may be embedded wholly or partially within the main body 20. Fully embedding or encapsulating the frame 130 within the main body 20 has the benefits of a simplified visual aesthetic, reduced biocompatibility requirements for the frame 130, and a lower risk of debonding between the frame 130 and the main body 20. Partially embedding the frame 130 within the main body 20, on the other hand, has the benefits of a simpler and more cost-effective method of manufacture, and offers greater colour variant options. However, partially embedding the frame 130 has a higher risk of debonding. Debonding may introduce areas (e.g. cracks and recesses) that are more difficult to clean hygienically.

Embedding the frame 130 within the main body 20 may be achieved by overmouling an elastomeric material onto the frame 130, in single or multiple operations. Alternatively, rolled sheets of an elastomeric material may be layered onto the inner surface, the outer surface or both surfaces of the frame and then compression moulding may be applied. Irrespective of the method of manufacture, the frame 130 may comprise anchor or interlock features (e.g., holes 138 or projections 139) and/or the surface of the frame 130 may be treated (e.g., plasma treatment) in order to aid bonding of the main body 20 to the frame 130.

In the examples illustrated in Figure 16, the frame 130 comprises a rib 135 for each segment 25 of the main body 20, which in this instance is six. In other examples, the frame 130 may comprise a fewer number of ribs 135; that is to say that the number of ribs 135 may be smaller than the number of segments 25. The stiffness of the frame 130, or rather the radial stiffness of the ribs 135, will depend on factors such as the choice of material for the frame 130, as well as the geometry (e.g. size and shape) of the ribs 135. In each of the examples illustrated in Figure 16, the frame 130 is formed of a plastic, such as PA, PE or PP, and has a flexural modulus of around 2.3 GPa. Additionally, each of the ribs 135 has a length of around 48 mm, a width of around 4.8 mm, and a thickness of around 0.8 mm. The frame then provides a relatively good balance between flexibility and support. However, it will be appreciated that alternative materials and geometries are possible.

As will now be explained, the provision of a frame 130 within the main body 20 has several potential benefits.

As noted above, the menstrual cup is removed by squeezing or pinching the main body 20. This then causes the main body 20 to fold inwardly, which in turn pulls the rim 30 away from the vaginal wall to break the seal between the cup and the vaginal wall. By providing a frame 130 within the main body 20, a user is able to squeeze or pinch the main body 20 much further down. Upon squeezing a lower part of the main body 20, the ribs 135 of the frame 130 bend inwardly relative to the base 134 and pull the rim 30 away from the vaginal wall to break the seal. The user is therefore able to break the seal by gripping the cup at a position much closer to the vaginal opening. The user is then presented with a more ergonomic process that reduces direct interaction with menstrual fluid on the vaginal wall.

The provision of a frame 130 increases the longitudinal stiffness of the cup, which in turn better allows the cup to be repositioned within the vagina without losing its shape. For example, contraction of the pelvic floor muscles, which may occur during bowel movement or exercise, will tend to push a menstrual cup further down in the vagina. A user may therefore wish to reposition the cup further up the vagina for increased reliability and comfort. By providing a frame 130 within the main body 20, a user is able to push the base of the cup with, for example, their index finger. The stiffness created by the frame 130 then causes to the cup to move rather than buckle and deform. As a result, the ergonomics and hygiene associated with repositioning the cup are improved over a conventional menstrual cup.

A further advantage of the frame 130 is that the resilience of the ribs 135 can be used to provide the necessary pressure to form a reliable seal between the rim 30 and the vaginal wall. As a result, the main body 20 may be formed of a softer elastomeric material, thereby improving user comfort. Conventional menstrual cups typically have a relatively high hardness and a relatively thick rim in an attempt to balance the conflicting requirements of reliability, comfort, and ease of use within a single material. A conventional menstrual cup may have a Shore hardness of between 40A and 70A. Cups at the lower, softer end of this scale may not have sufficient resilience to maintain a reliable seal. In particular, forces from the vaginal wall and pelvic floor may cause the cup to deform. Owing to the softness and pliability of the cup, the cup may fail to return to its intended shape within the vagina. A user may then be required to manually check and readjust the shape of the cup. Cups at the higher, harder end of this scale may be uncomfortable and may exert excessive pressure on the vaginal wall and/or the urethra. Additionally, owing to the higher hardness and stiffness, breaking the seal and removing the cup can be more difficult and uncomfortable. By providing a frame 130 within the main body 20, a softer elastomeric material may be employed for the main body 20 without compromising on sealing. A softer material leads to improved comfort and conformance with the vaginal wall, whilst the resilience provided by the frame 130 ensures that the cup retains a desired shape within the vagina during use. The main body 20 may be formed of an elastomeric material having a Shore hardness of between 30A and 50A, and yet the frame 130 ensures that the cup nevertheless maintains the desired shape. A Shore hardness of around 40A was found to provide a good balance between comfort and resilience, at least for the cups and frames illustrated in the Figures.

The frame 130 may also be used to support and/or house an electronic assembly (not shown) or components thereof. For example, the base 134 of the frame 130 illustrated in Figure 16(a) may be used to house an electronic assembly, such as a battery and printed circuit assembly. Additionally or alternatively, the ribs 135 may be used to support one or more sensors (e.g., temperature, optical or capacitive) of the electronic assembly. In some example, the sensor(s) may take the form of a strip that extends along the length of a rib 135. The provision of a frame 130 enables the electronic assembly to be securely mounted to the frame 130 and then encapsulated by the main body 20.

Should the menstrual cup include a stem that extends downwardly from the main body 20, the frame 130 may include a portion that projects into the stem to reinforce and stiffen the stem. As a result, a user may better grip the stem during removal of the cup.

Each of the menstrual cups described above may be inserted into the vagina by hand. However, the manner in which each cup collapses makes them ideally-suited for insertion using an applicator.

Figure 18 illustrates the use of an applicator with a menstrual cup. The applicator 150 is cylindrical in shape and comprises a plunger 151 that moves within an outer sleeve 152. In order to use the applicator 150, the base 21 of the cup 140 is inserted into the open end of the outer sleeve 152. The side wall 22 of the cup 140 is then squeezed and the cup 140 is pushed down into the outer sleeve 152. With the cup 140 located inside the applicator 150, the outer sleeve 152 may be inserted into the vagina and the plunger 1511 depressed in order to insert the cup 140 into the vagina.

The menstrual cups described herein have many potential advantages over conventional menstrual cups. For example, the cups have a relatively compact collapsed state that is relatively easy to form and hold. As a result, insertion of the cups into the vagina may be easier and more comfortable, particularly when used with an applicator. Additionally, the cup is better able to conform to vaginas of different sizes and shapes to provide an effective seal without undue discomfort. Moreover, the cup is able to react dynamically to changes in the size and shape of the vagina during use to provide improved sealing.

Whilst particular examples and embodiments have been described, it should be understood that various modifications may be made without departing from the scope of the invention as defined by the claims.

## Claims

1. A menstrual cup comprising a main body to hold menstrual fluid, wherein the main body comprises a plurality of segments, adjacent segments are connected along fold lines, the cup is moveable between an expanded state and a collapsed state, the main body folds along the fold lines when the cup moves between the expanded and collapsed states, and the segments project outwardly beyond the fold lines when the cup is in the expanded state, wherein the cup comprises a rim provided at an open end of the main body, and the rim has an outer edge having an elliptical profile in a radial direction when the cup is in the expanded state, **characterized in that** the outer edge is undulated in an axial direction when the cup is in the expanded state.

2. A menstrual cup as claimed in claim 1, wherein the segments are arranged about a central longitudinal axis, and the segments project radially outward beyond the fold lines when the cup is in the expanded state.

3. A menstrual cup as claimed in claim 1 or 2, wherein each of the fold lines has a maximum depth of at least 5 mm when the cup is in the expanded state.

4. A menstrual cup as claimed in any one of the preceding claims, wherein each of the fold lines has a depth that varies along the length of the main body.

5. A menstrual cup as claimed in any one of the preceding claims, wherein each of the fold lines has a depth that decreases towards a base of the main body.

6. A menstrual cup as claimed in any one of the preceding claims, wherein the cup comprises a central longitudinal axis and, when the cup is in the expanded state, each of the fold lines has a maximum radial distance of R1, each segment has a maximum radial distance of R2, and R2 is greater than R1, optionally wherein R2/R1 is at least 1.2, optionally wherein R2-R1 is at least 5 mm.

7. A menstrual cup as claimed in any one of the preceding claims, wherein, when the cup is in the expanded state, the main body has a fold angle of no greater than 130 degrees at each of the fold lines, optionally wherein the fold angle is no less than 60 degrees.

8. A menstrual cup as claimed in any one of the preceding claims, wherein an exterior surface of the main body undulates about a central longitudinal axis when the cup is in the expanded state.

9. A menstrual cup as claimed in claim 8, wherein the main body comprises a base, and a side wall upstanding from the base, the side wall comprises the plurality of segments, and the exterior surface of the side wall undulates about the longitudinal axis.

10. A menstrual cup as claimed in any one of the preceding claims, wherein the main body comprises a base and a side wall, the side wall comprises the plurality of segments, and the segments pivot relative to the base when the cup moves between the expanded and the collapsed states.

11. A menstrual cup as claimed in any one of the preceding claims, wherein the main body comprises between five and eight segments.

12. A menstrual cup as claimed in any one of the preceding claims, wherein the main body is resiliently biased to the expanded state.

13. A menstrual cup as claimed in any one of the preceding claims, wherein the rim folds as the cup moves between the expanded and collapsed states.

14. A menstrual cup as claimed in any one of the preceding claims, wherein the rim folds along fold lines that are aligned radially with the fold lines in the main body.

15. A menstrual cup as claimed in any one of the preceding claims, wherein the rim is funnelled towards the main body when the cup is in the expanded state.

## Patentansprüche

1. Menstruationstasse, umfassend einen Hauptkörper zum Aufnehmen von Menstruationsflüssigkeit, wobei der Hauptkörper mehrere Segmente umfasst, benachbarte Segmente entlang von Faltlinien verbunden sind, die Tasse zwischen einem ausgedehnten und einem zusammengefalteten Zustand bewegbar ist, der Hauptkörper sich entlang der Faltlinien faltet, wenn sich die Tasse zwischen dem ausgedehnten und dem zusammengefalteten Zustand bewegt, und die Segmente nach außen über die Faltlinien hinausragen, wenn sich die Tasse im ausgedehnten Zustand befindet, wobei die Tasse einen an einem offenen Ende des Hauptkörpers bereitgestellten Rand umfasst und der Rand eine Außenkante mit einem elliptischen Profil in radialer Richtung aufweist, wenn sich die Tasse im ausgedehnten Zustand befindet.
**dadurch gekennzeichnet, dass** der Außenrand im ausgedehnten Zustand des Btasses in axialer Richtung gewellt ist.

2. Menstruationstasse nach Anspruch 1, wobei die Segmente um eine zentrale Längsachse angeordnet sind und die Segmente radial nach außen über die Faltlinien hinausragen, wenn sich die Tasse im ausgedehnten Zustand befindet.

3. Menstruationstasse nach Anspruch 1 oder 2, wobei jede der Faltlinien eine maximale Tiefe von mindestens 5 mm aufweist, wenn sich die Tasse im ausgedehnten Zustand befindet.

4. Menstruationstasse nach einem der vorstehenden Ansprüche, wobei jede der Faltlinien eine Tiefe aufweist, die entlang der Länge des Hauptkörpers variiert.

5. Menstruationstasse nach einem der vorstehenden Ansprüche, wobei jede der Faltlinien eine Tiefe aufweist, die zu einer Basis des Hauptkörpers hin abnimmt.

6. Menstruationstasse nach einem der vorstehenden Ansprüche, wobei die Tasse eine zentrale Längsachse aufweist und, wenn sich die Tasse im ausgedehnten Zustand befindet, jede der Faltlinien einen maximalen radialen Abstand von R1 aufweist, jedes Segment einen maximalen radialen Abstand von R2 aufweist und R2 größer ist als R1, wobei optional R2/R1 mindestens 1,2 beträgt, wobei optional R2-R1 mindestens 5 mm beträgt.

7. Menstruationstasse nach einem der vorstehenden Ansprüche, wobei der Hauptkörper im ausgedehnten Zustand der Tasse an jeder der Faltlinien einen Faltwinkel von nicht mehr als 130 Grad aufweist, wobei der Faltwinkel optional nicht weniger als 60 Grad beträgt.

8. Menstruationstasse nach einem der vorstehenden Ansprüche, wobei eine Außenoberfläche des Hauptkörpers um eine zentrale Längsachse wellenförmig verläuft, wenn sich die Tasse im ausgedehnten Zustand befindet.

9. Menstruationstasse nach Anspruch 8, wobei der Hauptkörper eine Basis und eine von der Basis hochstehende Seitenwand umfasst, die Seitenwand mehrere Segmente umfasst und die Außenoberfläche der Seitenwand um die Längsachse wellenförmig verläuft.

10. Menstruationstasse nach einem der vorstehenden Ansprüche, wobei der Hauptkörper eine Basis und eine Seitenwand umfasst, die Seitenwand die Vielzahl von Segmenten umfasst und die Segmente relativ zur Basis schwenken, wenn sich die Tasse zwischen dem ausgedehnten und dem zusammengefalteten Zustand bewegt.

11. Menstruationstasse nach einem der vorstehenden Ansprüche, wobei der Hauptkörper zwischen fünf und acht Segmente umfasst.

12. Menstruationstasse nach einem der vorstehenden Ansprüche, wobei der Hauptkörper elastisch in den ausgedehnten Zustand vorgespannt ist.

13. Menstruationstasse nach einem der vorstehenden Ansprüche, wobei sich der Rand faltet, wenn sich die Tasse zwischen dem ausgedehnten und dem zusammengefalteten Zustand bewegt.

14. Menstruationstasse nach einem der vorstehenden Ansprüche, wobei der Rand entlang Faltlinien gefaltet wird, die radial mit den Faltlinien im Hauptkörper ausgerichtet sind.

15. Menstruationstasse nach einem der vorstehenden Ansprüche, wobei der Rand trichterförmig zum Hauptkörper hin verläuft, wenn sich die Tasse im ausgedehnten Zustand befindet.

## Revendications

1. Coupe menstruelle comprenant un corps principal pour contenir du fluide menstruel, dans laquelle le corps principal comprend une pluralité de segments, des segments adjacents sont reliés le long de lignes de pliage, la coupe est mobile entre un état déployé et un état replié, le corps principal se plie le long des lignes de pliage lorsque la coupe se déplace entre les états déployé et replié, et les segments font saillie vers l'extérieur, au-delà des lignes de pliage lorsque la coupe est à l'état déployé, dans laquelle la couple comprend un rebord fourni au niveau d'une extrémité ouverte du corps principal, et le rebord a un bord extérieur ayant un profil elliptique dans une direction radiale lorsque la coupe est à l'état déployé, **caractérisée en ce que** le bord extérieur est ondulé dans une direction axiale lorsque la coupe est à l'état déployé.

2. Coupe menstruelle selon la revendication 1, dans laquelle les segments sont agencés autour d'un axe longitudinal central, et les segments font saillie radialement vers l'extérieur au-delà des lignes de pliage lorsque la coupe est à l'état déployé.

3. Coupe menstruelle selon la revendication 1 ou 2, dans laquelle chacune des lignes de pliage a une profondeur maximale d'au moins 5 mm lorsque la coupe est à l'état déployé.

4. Coupe menstruelle selon l'une quelconque des revendications précédentes, dans laquelle chacune des lignes de pliage a une profondeur qui varie le long de la longueur du corps principal.

5. Coupe menstruelle selon l'une quelconque des revendications précédentes, dans laquelle chacune des lignes de pliage a une profondeur qui diminue vers une base du corps principal.

6. Coupe menstruelle selon l'une quelconque des revendications précédentes, dans laquelle la coupe comprend un axe longitudinal central et, lorsque la coupe est à l'état déployé, chacune des lignes de pliage a une distance radiale maximale de R1, chaque segment a une distance radiale maximale de R2, et R2 est supérieure à R1, éventuellement, dans laquelle R2/R1 est d'au moins 1,2, éventuellement, dans laquelle R2 - R1 est d'au moins 5 mm.

7. Coupe menstruelle selon l'une quelconque des revendications précédentes, dans laquelle, lorsque la coupe est à l'état déployé, le corps principal présente un angle de pliage ne dépassant pas 130 degrés au niveau de chacune des lignes de pliage, éventuellement, dans laquelle l'angle de pliage n'est pas inférieur à 60 degrés.

8. Coupe menstruelle selon l'une quelconque des revendications précédentes, dans laquelle une surface extérieure du corps principal ondule autour d'un axe longitudinal central lorsque la coupe est à l'état déployé.

9. Coupe menstruelle selon la revendication 8, dans laquelle le corps principal comprend une base, et une paroi latérale s'élevant à partir de la base, la paroi latérale comprend la pluralité de segments, et la surface extérieure de la paroi latérale ondule autour de l'axe longitudinal.

10. Coupe menstruelle selon l'une quelconque des revendications précédentes, dans laquelle le corps principal comprend une base et une paroi latérale, la paroi latérale comprend la pluralité de segments, et les segments pivotent par rapport à la base lorsque la coupe passe de l'état déployé à l'état replié.

11. Coupe menstruelle selon l'une quelconque des revendications précédentes, dans laquelle le corps principal comprend entre cinq et
huit segments.

12. Coupe menstruelle selon l'une quelconque des revendications précédentes, dans laquelle le corps principal est élastiquement incliné vers l'état déployé.

13. Coupe menstruelle selon l'une quelconque des revendications précédentes, dans laquelle le rebord se plie lorsque la coupe passe de l'état déployé à l'état replié.

14. Coupe menstruelle selon l'une quelconque des revendications précédentes, dans laquelle le rebord se plie le long de lignes de pliage alignées radialement sur les lignes de pliage dans le corps principal.

15. Coupe menstruelle selon l'une quelconque des revendications précédentes, dans laquelle le rebord est canalisé vers le corps principal lorsque la coupe est à l'état déployé.
